# EUROPEAN PATENT APPLICATION

(11) **EP 4 485 471 A1**
(43) Date of publication of application: **01.01.2025**
(21) Application number: 24183442.3
(22) Date of filing: 20.06.2024
(51) Int. Cl.: G16H 20/13, A61J 1/03, A61J 7/00, G06Q 10/0833, G06Q 10/10, G06T 7/00

(54) **VERIFICATION SYSTEM FOR A BLISTER CARD PACKAGING SYSTEM**

(30) Priority: 21.06.2023 US 202363509365 P; 12.06.2024 US 202418741444
(71) Applicant: RXSAFE LLC, Vista, CA 92081 (US)
(72) Inventor: HOLMES, William K., 92081 Vista (US); GAASCH, Tom F., 92081 Vista (US); ARSENAULT, Aaron, 92081 Vista (US)
(74) Representative: HGF

(57) **Abstract**

A verification system for a blister card packaging system. The verification system includes an electronic processor. The electronic processor is configured to capture a successive plurality of images of a blister of a blister card, display an image of at least a portion of the blister card for verification, receive a selection of the blister subsequent to the display of the image, and display the successive plurality of images in response to receiving the selection.

## Description

### CROSS-REFERENCE TO RELATED APPLICATIONS

This application claims priority to U.S. Provisional Patent Application No. 63/509,365, filed June 21, 2023, the entire contents of which are incorporated by reference herein.

### FIELD

The present disclosure relates to blister card packaging systems. More particularly, the present disclosure relates to verification systems for blister card packaging systems.

### SUMMARY

Pharmacies use several types of packaging to provide pharmaceutical products or medications to consumers. The types of packaging may include strip packages, blister cards, pill vials, and the like. Pill vials and strip packages are most appropriate for individual customers of retail pharmacies. However, institutional customers, for example, nursing homes, palliative or hospice facilities, hospitals, and the like use blister cards extensively.

Currently most blister cards are manually packaged by a pharmacist or pharmacy technician. Verification of the blister cards is also performed manually during packaging. Automated or semi-automated blister card packagers do not have a robust verification system for verifying that a blister card is correctly packaged. Rather, the pharmacist needs to manually investigate the packaged blister cards before dispensing. Some automatic packagers may verify pharmaceuticals at an intermediate step prior to the pharmaceuticals are dispensed into the blister card. These automatic packagers use a puck in the intermediate step to temporarily hold the pharmaceuticals while a camera captures images of the pharmaceuticals prior to being packaged in the blister card. Some of the disadvantages of such an intermediate verification system is that the puck may have debris from prior pharmaceuticals that can result in cross-contamination. Additionally, since the images are captured before the pharmaceuticals are dispensed to the blister card, the pharmacist may still need to verify the final package to make sure all the pills from the puck are transferred to the blister card.

Accordingly, there is a need for a verification system that cures the above described deficiencies of intermediate verification systems.

One embodiment provides a verification system for a blister card packaging system. The verification system includes an electronic processor. The electronic processor is configured to capture a successive plurality of images of a blister of a blister card, display an image of at least a portion of the blister card for verification, receive a selection of the blister subsequent to the display of the image, and display the successive plurality of images in response to receiving the selection.

Another embodiment provides a method for verification of a blister card. The method includes capturing a successive plurality of images of a blister of a blister card, displaying an image of at least a portion of the blister card for verification, receiving a selection of the blister subsequent to the display of the image, and displaying the successive plurality of images in response to receiving the selection.

Yet another embodiment provides a blister card packaging system, the blister card packaging system includes an electronic processor. The electronic processor is configured capture a successive plurality of images of a blister of a blister card, display an image of at least a portion of the blister card for verification, receive a selection of the blister subsequent to the display of the image, and display the successive plurality of images in response to receiving the selection.

### BRIEF DESCRIPTION OF THE DRAWINGS

FIG. 1 illustrates a blister card in accordance with some examples.
FIG. 2 is a perspective view of an automatic blister card packager in accordance with some examples.
FIG. 3 is an enlarged view of a portion of the automatic blister card packager of FIG. 2 in accordance with some examples.
FIG. 4 is a front perspective view of a universal feed cassette of the automatic blister card packager of FIG. 2 with a part of a housing removed in accordance with some examples.
FIG. 5 is a plan view of the universal feed cassette of FIG. 4 in accordance with some examples.
FIG. 6 is a perspective view of the cartridge and the cartridge mechanism of the universal feed cassette of FIG. 4 in accordance with some examples.
FIG. 7 is an enlarged view of a portion of the automatic blister card packager of FIG. 2 in accordance with some examples.
FIG. 8 is a perspective view of the automatic blister card packager of FIG. 2 in accordance with some examples.
FIG. 9A and 9B are perspective views of a packaging unit of the automatic blister card packager of FIG. 2 in accordance with some examples.
FIG. 10 is a perspective view of the packaging unit of FIGS. 9A and 9B in accordance with some examples.
FIG. 11 is a perspective view of a packaging equipment of the packaging unit of FIGS. 9A-10 in accordance with some examples.
FIG. 12 is a perspective view of a packaging plate of the packaging equipment of FIGS. 9A-10 in accordance with some examples.
FIG. 13 is a block diagram of a verification system used with the automatic blister card packager of FIG. 2 in accordance with some examples.
FIG. 14 is a flowchart of a method of verification for a blister card in accordance with some examples.
FIG. 15 illustrates a verification screen generated by the verification system of FIG. 13 in accordance with some examples.
FIGS. 16A and 16B illustrate a verification screen generated by the verification system of FIG. 13 in accordance with some examples.
FIG. 17 illustrates a verification screen generated by the verification system of FIG. 13 in accordance with some examples.
FIG. 18 illustrates a verification screen generated by the verification system of FIG. 13 in accordance with some examples.

### DETAILED DESCRIPTION

Before any examples, features, aspects, or embodiments are explained in detail, it is to be understood that the examples, features, aspects, or embodiments are not limited in their application to the details of construction and the arrangement of components set forth in the following description or illustrated in the following drawings. The examples, features, aspects, or embodiments are capable of being practiced or of being carried out in various ways.

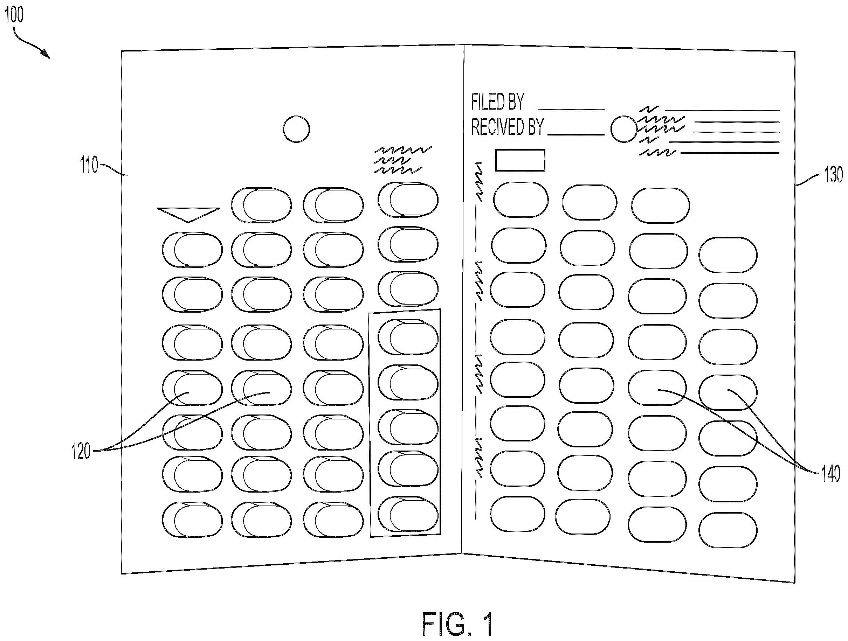

FIG. 1 illustrates an example of a blister card 100. The blister card 100 includes a blister sheet 110 having a one or more of blister compartments 120, for example, twenty-eight to thirty-two depending on the configuration of the blister card 100. Each blister compartment 120 may receive a single unit of medication or may receive more than a single unit of medication. When the blister compartments 120 are filled, a label sheet 130 is attached to the back of the blister sheet 110 to seal the blister compartments 120. The label sheet 130 includes information regarding the blister card 100 including, for example, a facility name, a patient name, a medication name, a medication dose, and the like. The label sheet 130 includes seal portions 140 aligned with each blister compartment 120 such that a user may tear the seal portions 140 to retrieve the packaged medication.

In the example illustrated in FIG. 1, the blister card 100 is a standard 6"x9" card. In other examples, the blister card 100 may be other sizes. The blister card 100 may be used for filling different amounts of pills for different periods of time. For example, the blister card 100 may be used for one week of pills (e.g., 7 pills), for up to four weeks or a month of pills (e.g., 28-31 pills), or more. In some examples, the blister card 100 is a "pre-pack" having only one medication in some or all the blister compartments 120. In other examples, the blister card 100 may include more than one pill in the same blister compartment 120 or distributed among the different blister compartments 120. In the example illustrated, the label sheet 130 is shown attached to the blister sheet 110; however, the label sheet 130 may be separately provided from the blister sheet 110. The label sheet 130, whether provided with the blister sheet 110 or separate from the blister sheet 110, may be cold sealed or hot sealed with the blister sheet 110 after filling. In some examples, a bar code may be printed on the label sheet 130 to provide identification information. The bar code may be scanned to retrieve information regarding the blister card 100 as provided above. The bar code may be pre-printed before filling or may be printed after filling to uniquely identify the blister card 100. This bar code may be later used by a filling machine to identify the blister card 100 currently being filled.

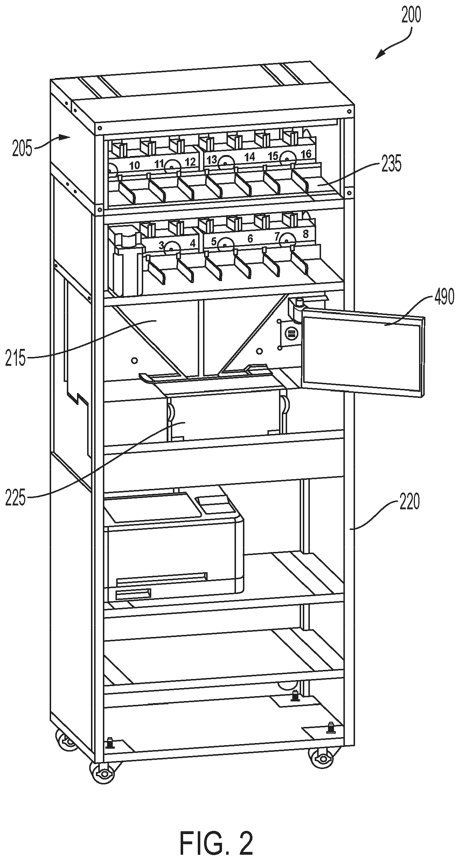

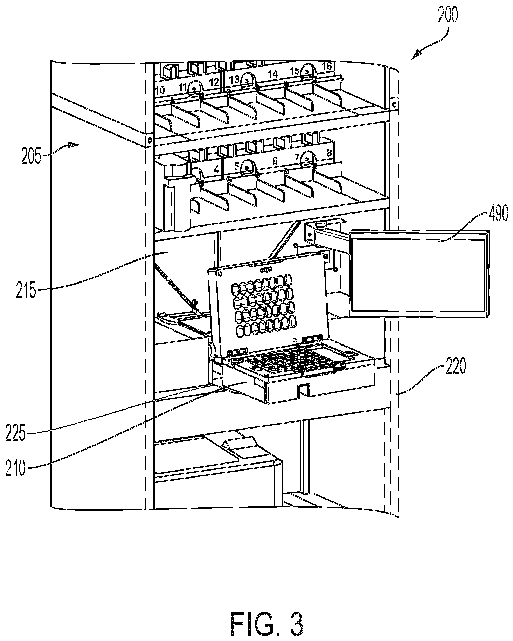

FIGS. 2-3 illustrate an example of an automatic blister card packager 200 that efficiently packages the blister cards 100. In the example illustrated, the automatic blister card packager 200 includes a universal feed cassette 205, a packaging unit 210, and a manifold 215 that connects the universal feed cassette 205 and the packaging unit 210. The universal feed cassette 205 and the packaging unit 210 are supported by a frame 220. The universal feed cassette 205 receives medications from the bulk canisters and individually dispenses pills to the packaging unit 210. The automatic blister card packager 200 includes a door 225 that provides selective access to the packaging unit 210.

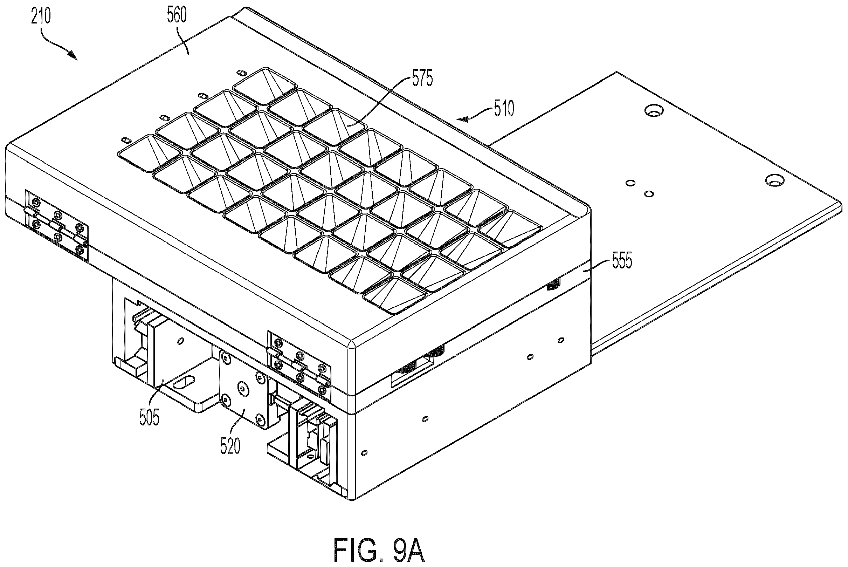

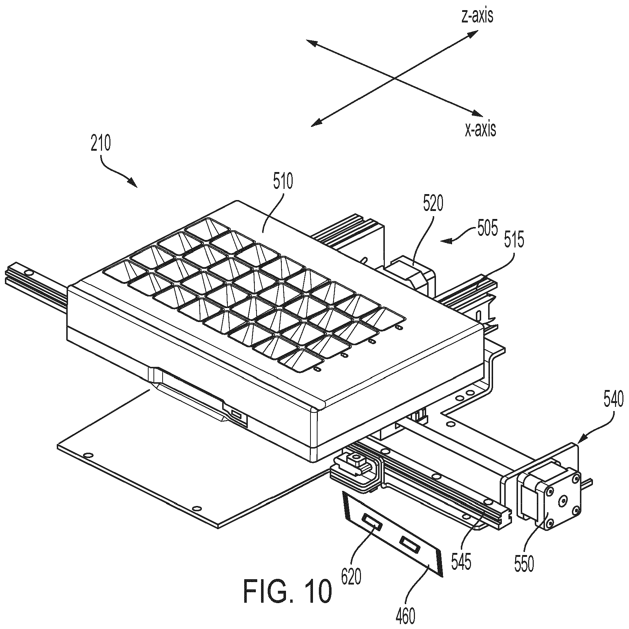

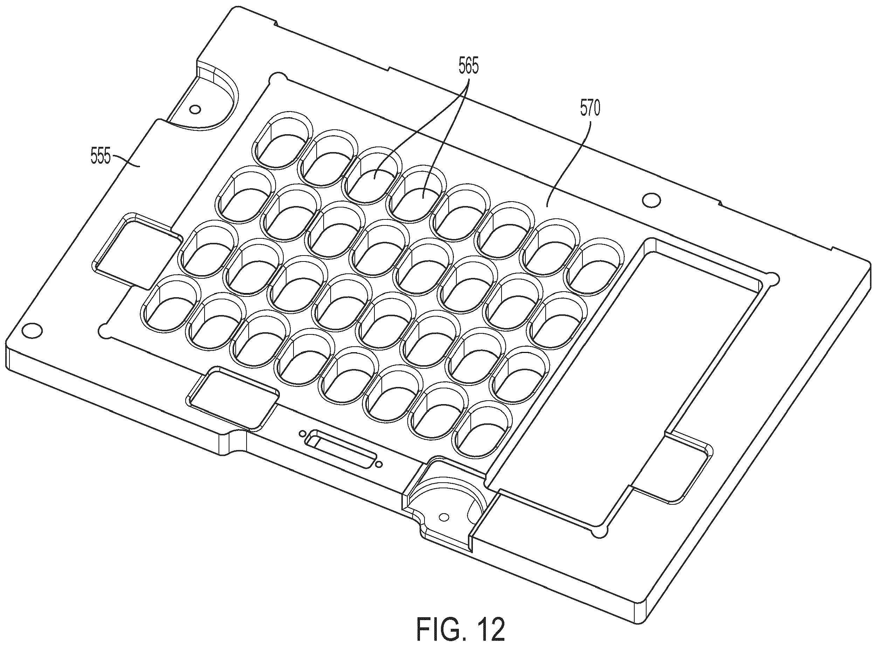

As shown in FIGS. 2-3, the packaging unit 210 is movable toward and away from the automatic blister card packager 200. This movement is generally in a forward-backward direction along a first axis. The first axis may also be referred to as a z-axis. In the illustrated example, however, the packaging unit 210 is also movable side-to-side within the automatic blister card packager 200, as shown in FIGS. 12-14. This movement is along a second axis that is perpendicular to the first axis. The second axis may also be referred to as an x-axis. Movement along the first axis is controlled by a first rail system 505 (shown in FIGS. 9A-10). Movement along the second axis is controlled by a second rail system 540 (shown in FIG. 10). The packaging unit 210 moves forward-backward along the first axis and side-to-side along the second axis to position different openings beneath the manifold 215, as further described below.

An example blister card packaging machine is described in U.S. Patent Application No. 17/916,011 filed on September 29, 2022, entitled "AUTOMATIC PACKAGER FOR MEDICATIONS," the entire contents of which are hereby incorporated by reference.

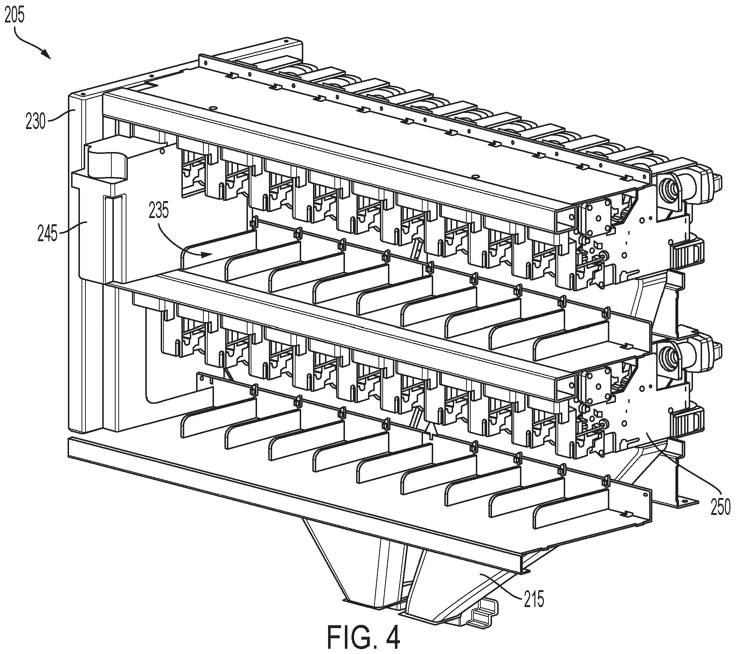

Referring to FIGS. 4-5, the universal feed cassette 205 includes a housing 230 having a plurality of cartridge slots 235 within the housing 230. Dispensing openings 240 are provided on the bottom of the housing 230. The dispensing openings 240 are in communication with the manifold 215 of the automatic blister card packager 200. In the example illustrated in FIGS. 4-5, the universal feed cassette 205 includes up to sixteen cartridge slots 235. In some examples, the cartridge slots 235 are arranged in a duplex-formation (e.g., two vertically-stacked rows) such that a second row of cartridge slots 235 are provided above a first row of cartridge slots 235 within the housing 230 to facilitate faster filling of blister cards 100.

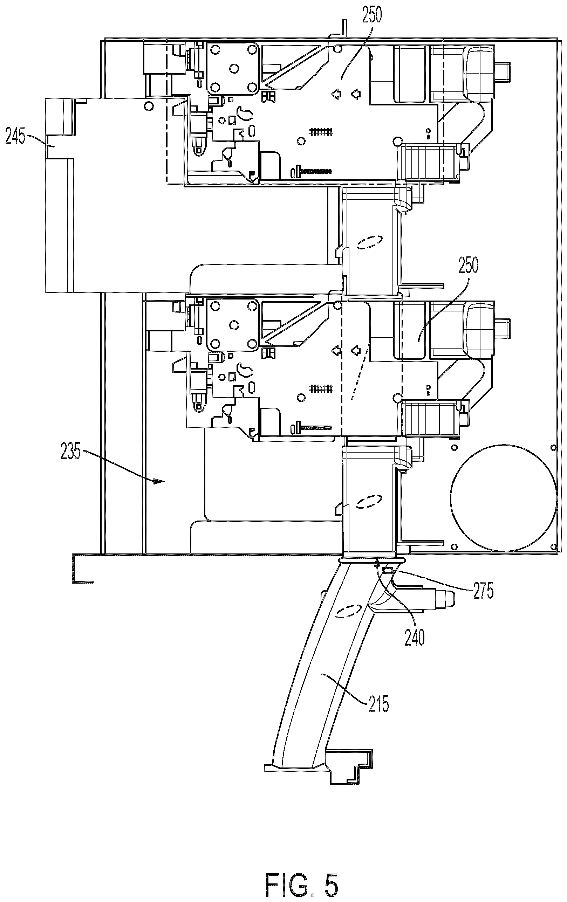

FIG. 5 illustrates a side-profile view of the duplex-formation of the cartridge slots 235. In some examples, the universal feed cassette 205 may only include a single row of cartridge slots 235, or may include more than two rows of cartridge slots 235. The cartridge slots 235 are configured to receive cartridges 245. A plurality of cartridge mechanisms 250, one for each cartridge slot 235 is fixed to the housing 230. When received in the cartridge slots 235, the cartridges 245 are connected to the cartridge mechanism 250. The cartridge mechanism 250 individually dispenses medications from the cartridge 245 as described below. The dispensing openings 240 transfer the medications from the cartridges 245 to the packaging unit 210 for packaging.

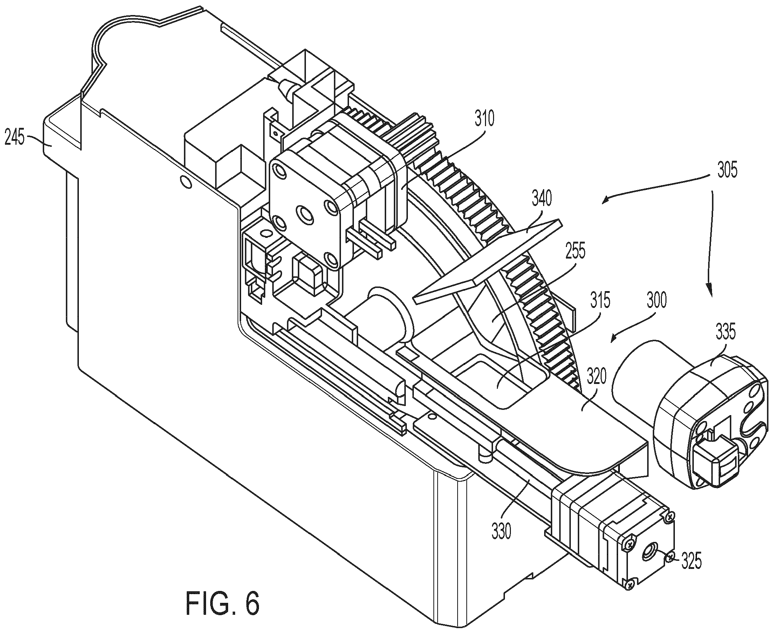

Referring to FIG. 6, the cartridge 245 stores medications during the dispensing process and includes a wheel 255 to transport the medications to the cartridge mechanism 250. The cartridge mechanism 250 includes a shuttle system 300, a camera system 305, and a motor assembly 310. The wheel 255 of the cartridge 245 is driven by the motor assembly 310. When the wheel 255 is driven, the wheel 255 singulates medications stored in the cartridge 245 and delivers the singulated medications to the shuttle system 300. The shuttle system 300 includes a platform 315, a shuttle 320, and a shuttle drive 325. The platform 315 may be made from a clear or translucent plastic material. An LED lighting system may be provided over and/or under the platform 315 to illuminate the contents on the platform 315 when a camera system 305 of the cartridge mechanism 250 is capturing an image of the contents. The LED lighting system may emit visible or infrared light to illuminate the platform 315.

The shuttle 320 may be moved laterally between the platform 315, over a medication reservoir of the cartridge 245, and over a conduit 330. The shuttle 320 transfers the medications from the platform 315 either back to the cartridge 245 or to the conduit 330. The shuttle 320 is driven by the shuttle drive 325. The shuttle drive 325 may be a motor assembly, an actuator, or the like.

The camera system 305 includes a camera 335 and a mirror 340. The camera 335 is positioned at the back of the cartridge mechanism 250. The camera 335 may be a still camera or a video camera that captures an image of the contents of the platform. The mirror 340 is placed directly above the platform 315 and is tilted at a 45-degree angle such that the camera 335 positioned at the back of the cartridge mechanism 250 can capture an image of the platform 315. In other examples, the camera 335 may be placed directly above the platform or in other configurations with or without using the mirror 340.

An example cartridge 245 and cartridge mechanism 250 are described in U.S. Patent Application No. 16/160,535 filed on October 15, 2018, entitled "UNIVERSAL FEED MECHANISM FOR AUTOMATIC PACKAGER," the entire contents of which are hereby incorporated by reference.

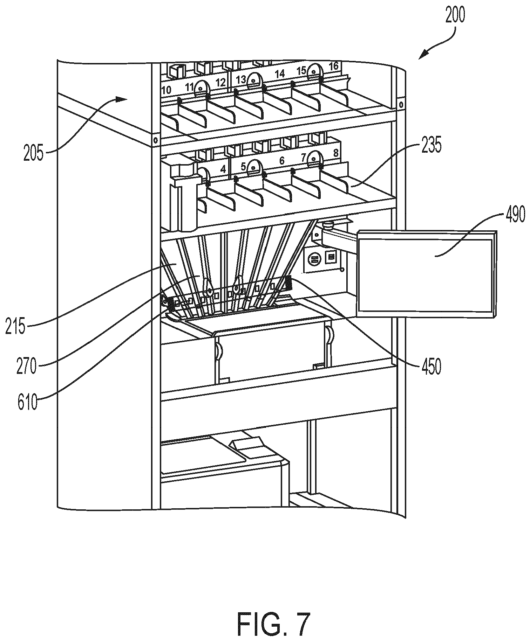

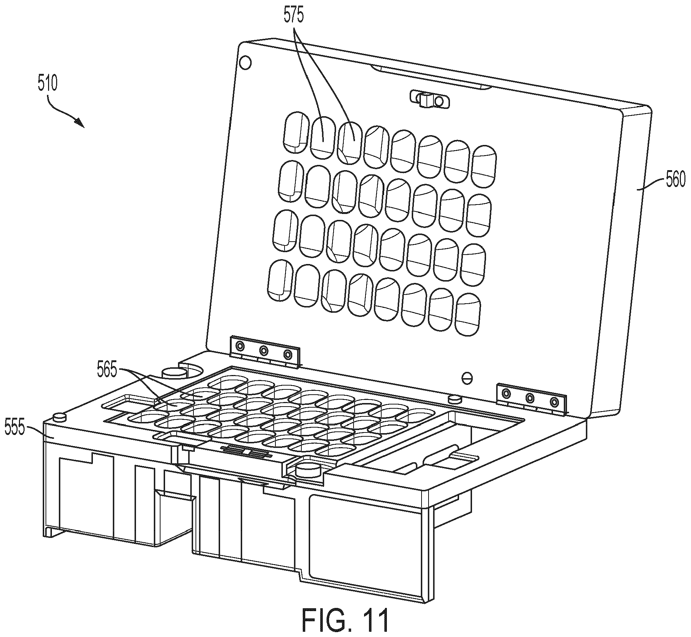

FIG. 7 illustrates one example of the manifold 215 for use with the automatic blister card packager 200. The manifold 215 helps direct pharmaceuticals (e.g., pills, vitamins, etc.) from the universal feed cassette 205 to the packaging unit 210. In the illustrated example, the manifold 215 includes eight separate tracks or channels 270. The number of channels 270 correspond to the number of cartridge slots 235 (and thereby cartridges) in each row of the universal feed cassette 205. As such, in some examples, the manifold 215 may include fewer or more channels 270. Each channel 270 has a separate outlet that corresponds to one of the openings 575 (shown in FIG. 11) in the packaging unit 210. When pharmaceuticals are delivered from the universal feed cassette 205, the pharmaceuticals travel through the channels 270 of the manifold 215, through the outlets, and into one of the openings 575 in the packaging unit 210.

Using the manifold 215, the cartridges 245 may simultaneously (or relatively simultaneously) release pills to fill a row (or a column) of the blister compartments 120, the packaging unit 210 may index to the next row (or the next column) of the blister compartments 120 along the first axis (i.e., the z-axis)(or along the second axis (i.e., the x-axis)), and the process may repeat until all of the rows (or columns) of the blister compartments 120 are filled. In some examples, the cartridges 245 may release a single pill into each of the blister compartments 120. In other examples, the cartridges 245 may release multiple pills into each of the blister compartments 120 (e.g., between two and eight pills, depending on the size of the blister compartment 120).

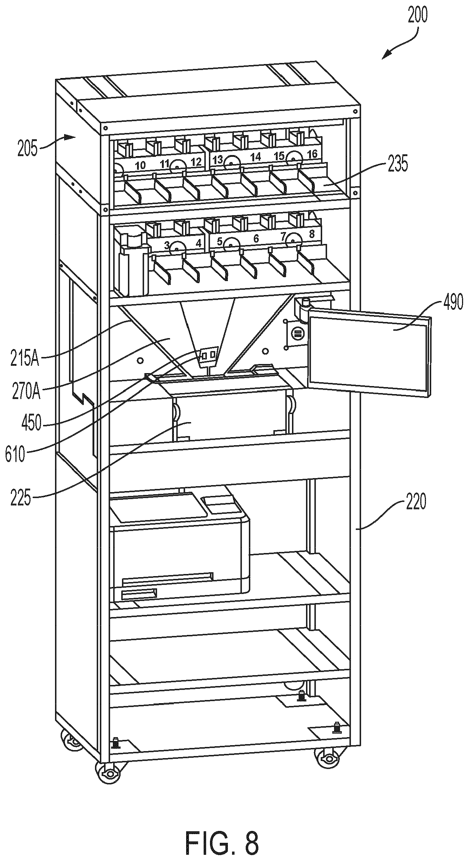

FIG. 8 illustrates another example of a manifold 215A for use with the automatic blister card packager 200. In the illustrated example, the manifold 215A includes two shared tracks or channels 270A that dispense pharmaceuticals through shared outlets to the packaging unit 210. For example, four cartridge slots 235 (and thereby cartridges 245) in one row of the universal feed cassette 205 share one half of the manifold 215A (that is, a first channel 270A) to dispense pharmaceuticals through a first outlet, while four other cartridge slots 235 (and thereby cartridges 245) in the same row of the universal feed cassette 205 share the other half of the manifold 215A (that is, a second channel 270A) to dispense pharmaceuticals through a second outlet. In other examples, the manifold 215A may include a single shared track or channel that dispenses pharmaceuticals through a single outlet. The illustrated example of the manifold 215, therefore, can only direct pharmaceuticals to two openings 575 (shown in FIG. 12) of the packaging unit 210 at a time. The packaging unit 210, however, can move side-to-side to align with different openings 575 in a row of openings 575 with the shared outlets of the manifold 215A.

Using the manifold 215A, only two of the cartridges 245 (e.g., one of the cartridges 245 associated with each shared channel 270A) may release pills. The pills are then delivered by the shared outlets into the associated ones of the blister compartments 120 of the blister card 100. After the associated ones of the blister compartments 120 in a row are filled, the packaging unit 210 may then move along the second axis (i.e., the x-axis) to fill additional blister compartments 120 in the row. Once a complete row of blister compartments 120 is filled, the packaging unit 210 may index to the next row of blister compartments 120 along the first axis (i.e., the z-axis), and the process may repeat. Such a process may be used to deliver a single pill to each blister compartment 120 of the blister card 100. Alternatively, multiple pills may be delivered to each blister compartment 120 of the blister card 100. For example, more than one cartridge 245 associated with each shared channel 270A may simultaneously (or relatively simultaneously) release a pill into the shared channel 270A to deliver multiple pills to a single blister compartment 120. Alternatively, each cartridge 245 may release more than one pill into the shared channel 270A. In some examples, a complete row of blister compartments 120 may not be filled before the packaging unit 210 moves on to the next row. Rather, the packaging unit 210 may "zig-zag" along the first and second axes to fill the blister compartments 120.

Referring to FIG. 5, cameras 275 are mounted at or near the junction of the conduits and the manifold 215. Each camera 275 is associated with one of the cartridge 245 supported in the universal feed cassette 205. The cameras 275 are operable to capture images and determine whether the proper number and/or type of pharmaceuticals are being dispensed from the cartridges 245. The cameras 275 capture images of pharmaceuticals exiting the universal feed cassette 205 and compare features (e.g., color, contour, size, shape, inscription, etc.) of the pharmaceuticals to stored images of known pharmaceuticals. In some examples, recognition software may be employed to automatically compare the images captured by the cameras to stored images. In other examples, the captured images may be transmitted to a remotely-located pharmacist or technician who analyzes the images and verifies that the correct number and type of pharmaceuticals were dispensed. In further examples, the cameras 275 may be infrared sensors that only detect whether an object (e.g., a pill) drops through the universal feed cassette 205, rather than identifying the particular type of pharmaceutical.

Referring to FIGS. 9A-9B, the packaging unit 210 includes a first rail system 505, and packaging equipment 510. The first rail system 505 includes first rails 515 and a first rail drive 520 and moves the packaging equipment 510 along a Z-axis of the automatic blister card packager 200 (see also FIG. 10). In the example illustrated, the first rails 515 include fixed rails 525, first moveable rails 530, and second moveable rails 535. The fixed rails 525 are stationary and start at a rear of the packaging unit 210 and extend, for example, up to a third of the length of the packaging unit 210. The first moveable rails 530 slide along the fixed rails 525 and extend the first rails 515 up to two thirds of the length of the packaging unit 210. The second moveable rails 535 slide along the first moveable rails 530 and extend the first rails 515 up to the entire length of the packaging unit 210. The packaging equipment 510 is attached to the second moveable rails 535 to move with the second moveable rails 535. The first rails 515 telescope the packaging equipment 510 along the length of the packaging unit 210. The first rail drive 520 may be a motor assembly, an actuator, or the like that drives the packaging equipment 510 using the first rails 515.

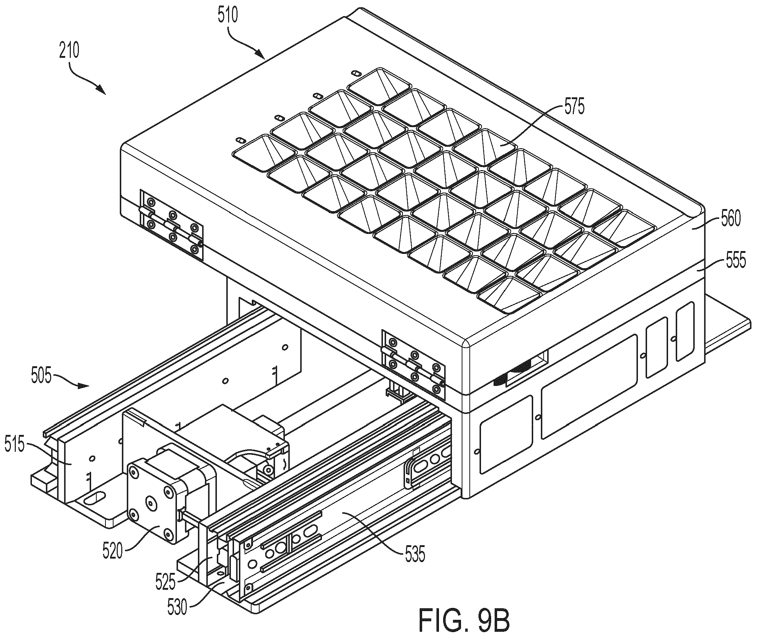

Referring to FIG. 9A, when in the first position, the packaging equipment 510 is within the automatic blister card packager 200 and is inaccessible by a pharmacist and/or technician. The first rail drive 520 moves the packaging equipment 510 under the manifold 215 within the automatic blister card packager 200 to fill a blister card 100 placed in the packaging equipment 510. Referring to FIG. 9B, when in the second position, the packaging equipment 510 may still be within the automatic blister card packager 200. However, at least a portion of the packaging equipment 510 is accessible by a user to pull the packaging equipment. When in the second position, a user may pull the packaging equipment 510 towards the user.

Referring to FIG. 10, the packaging unit 210 also includes a second rail system 540. The second rail system 540 includes second rails 545 and a second rail drive 550 and moves the packaging equipment 510 and the first rail system 505 along an X-axis of the automatic blister card packager 200. The second rails 545 and the second rail drive 550 work similar as the first rails 515 and the first rail drive 520 to move the packaging equipment 510 and the first rail system 505 along the X-axis. Alternatively, in some examples, the second rail system 540 moves the packaging equipment 510 and the first rail system 505 drives the packaging equipment 510 and the second rail system 540. In yet other examples, different drive configurations may be used to move the packaging equipment within and in and out of the automatic blister card packager 200.

Referring to FIG. 11-12, the packaging equipment 510 includes a packaging plate 555 and a packaging lid 560. The packaging plate 555 includes blister openings 565 provided in a blister card receiving portion 570. The blister openings 565 accommodate the blister compartments 120 of the blister sheet 110. In the example illustrated, the blister openings 565 are through-holes that extend through the packaging plate 555. The packaging lid 560 is hingedly connected to the packaging plate 555. The packaging lid 560 includes a top side and a bottom side opposite the top side. Medication receiving openings 575 extend from the top side to the bottom side of the packaging lid 560.

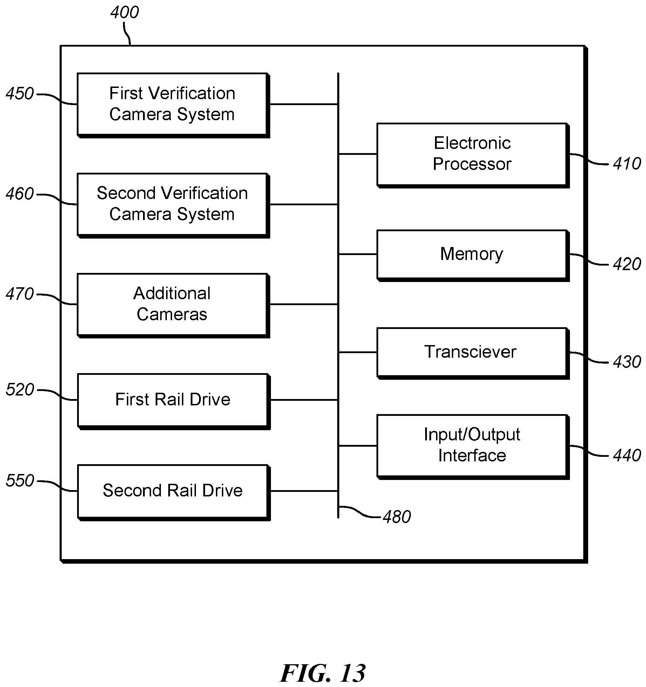

FIG. 13 is a block diagram of an example verification system 400 for the automatic blister card packager 200. In the example illustrated, the verification system 400 includes an electronic processor 410, a memory 420, a transceiver 430, and input/output interface 440, a first verification camera system 450, a second verification camera system 460, additional cameras 470, the first rail drive 520, and the second rail drive 550. The electronic processor 410, the memory 420, the transceiver 430, the input/output interface 440, the first verification camera system 450, the second verification camera system 460, additional cameras 470, the first rail drive 520, and the second rail drive 550 communicate over one or more control and/or data buses (e.g., a communication bus 480). In some examples, the verification system 400 is part of the automatic blister card packager 200 and resides within the frame 220. In other examples, the verification system 400 may be a separate device that is communicatively coupled with the automatic blister card packager 200. In these examples, the components of the verification system may be distributed between the frame 220 and the separate device. The verification system 400 may be used with other blister card packagers and may include more or fewer components than those illustrated in FIG. 13 depending on the blister card packager. In some examples, the verification system 400 may be used independently of or interoperably with the automatic blister card packager 200 or other blister card packagers.

In some examples, the electronic processor 410 is implemented as a microprocessor with separate memory, such as the memory 420. In other examples, the electronic processor 410 may be implemented as a microcontroller (with the memory 420 on the same chip). In other examples, the electronic processor 410 may be implemented using multiple processors. In addition, the electronic processor 410 may be implemented partially or entirely as, for example, a field-programmable gate array (FPGA), an applications specific integrated circuit (ASIC), and the like, and the memory 420 may not be needed or be modified accordingly. In the example illustrated, the memory 420 includes non-transitory, computer-readable memory that stores instructions that are received and executed by the electronic processor 410 to carry out the functionality of the verification system described herein. The memory 420 may include, for example, a program storage area and a data storage area. The program storage area and the data storage area may include combinations of different types of memory, such as read-only memory and random-access memory.

The transceiver 430 enables wired or wireless communication between the verification system 400 and other devices over one or more communication networks. In other examples, the transceiver 430 may include separate transmitting and receiving components, for example, a transmitter and a receiver.

The input/output interface 440 may include one or more input mechanisms (e.g., a touch screen, a keypad, a button, a knob, and the like), one or more output mechanisms (e.g., a display, a printer, a speaker, and the like), or a combination thereof. The input/output interface 440 receives input from the input devices actuated by a user, and provides output to the output devices with which a user interacts. In one example, the input/output interface 440 includes a display 490 (shown in FIGS. 2-3) configured to display images captured and processed by the verification system 400.

The first verification camera system 450 and the second verification camera system 460 are configured to capture images of the blister card 100 during and/or after filling of the blister card 100. The first verification camera system 450 and the second verification camera system 460 are configured to capture the images from opposing sides of the blister card 100 and the blister compartments 120. For example, the first verification camera system 450 includes one or more first verification cameras 610 (see FIGS. 7-8) configured to capture images of the contents of the blister compartments 120 from the open end of the blister compartments 120 (e.g., from above the blister compartments 120). The second verification camera system 460 includes one or more second verification cameras 620 (see FIGS. 7-8) configured to capture images of the contents of the blister compartments 120 from the closed end of the blister compartments 120 (e.g., from below the blister compartments 120). To capture the images of the blister compartments 120, the first verification camera system 450 and the second verification camera system 460 may be placed in the automatic blister card packager 200 adjacent the packaging unit 210 as further described below. In some examples, the first verification camera system 450 and the second verification camera system 460 may together be referred to as a camera system 450, 460.

Referring to FIG. 7, the first verification camera system 450 may be placed near the manifold 215. The first verification camera system 450 may include a plurality of first verification cameras 610 placed near the junction of the manifold 215 and the packaging unit 210. The plurality of first verification cameras 610 can include one first verification camera 610 for each track of the manifold 215. The first verification camera 610 for each track may be placed just above the junction of the manifold 215 and the packaging unit 210. The first verification camera 610 for each track is configured to capture an image of the blister compartment 120 corresponding to the track from above the blister compartment 120. The first verification camera 610 may capture the images before and/or after one or more pills are released into the blister compartment 120. In some examples, the first verification camera system 450 and the corresponding plurality of first verification cameras 610 may be placed in other locations. In one example, the tracks of the manifold 215 may curve backward toward the bottom end of the manifold 215 to direct the medications into the blister compartments 120. The first verification camera system 450 may be provided just above the curved portion and behind the manifold 215 such that the plurality of first verification cameras 610 have a view of the of the blister compartments through the curved portions of the track. In some examples, the first verification camera system 450 may include more or fewer first verification cameras 610 than the number of tracks in the manifold 215. In these examples, the first verification cameras 610 or the packaging unit 210 may be moved to capture the images from above the blister compartments 120.

Referring to FIG. 8, the first verification camera system 450 may be placed near the manifold 215A. In this example, the first verification camera system 450 may include two first verification cameras 610, one for each track 270A of the manifold 215A. Alternatively, the first verification system 450 may include a single verification camera that has visibility to both tracks 270A. The tracks 270A include a curve near the bottom end of the manifold 215A. The tracks 270A curve inward from the manifold 215A. The first verification cameras 610 are placed near the junction of the manifold 215A and the packaging unit 210 and just above the curved portion of the tracks 270A such that the first verification cameras 610 have a view of the blister compartments 120 through the curved portions of the tracks 270A. The first verification cameras 610 are configured to capture an image of the blister compartment 120 corresponding to the track 270A from above the blister compartment 120. The first verification cameras 610 may capture the images before and/or after one or more pills are released into the blister compartment 120. In some examples, the first verification camera system 450 and the corresponding plurality of first verification cameras 610 may be placed in other locations.

Referring to FIG. 10, the second verification camera system 460 may be placed in the automatic blister card packager 200 under the packaging unit 210. The second verification camera system 460 may include one or more second verification cameras 620 configured to capture images of the blister compartments 120 from under the packaging equipment 510. The first verification camera system 450 and the second verification camera system 460 are therefore configured to capture images of the blister compartments 120 from the top and the bottom of the blister card 100 during and/or after filling of the blister card 100. In some examples, the second verification camera system 460 and the corresponding one or more second verification cameras 620 may be placed in other locations, for example, on an opposite side of packaging equipment 510.

In some examples, the first verification camera system 450 and the second verification camera system 460 may also include integrated lights (e.g., flash lights) to illuminate the blister compartments 120 when the images are taken. These lights may be activated when the cameras 610, 620 are capturing images of the blister compartments 120. In some examples, the first verification camera system 450 is configured to capture images of individual blister compartments 120 from above during filling, whereas the second verification camera system 460 is configured to capture an image of the filled blister card 100 as a whole from below after filling. In other examples, both the first verification camera system 450 and the second verification camera system 460 are configured to capture images of individual blister compartments 120, from above and below respectively, during filling. In still other examples, both the first verification camera system 450 and the second camera verification system 460 are configured to capture images of the blister card 100 as a whole from above and below. In still other examples, both the first verification camera system 450 and the second camera verification system 460 are configured to capture images of portions (for example, rows or columns) of the blister card 100 from above and below. The additional cameras 470 include the cameras 275 and the camera system 305.

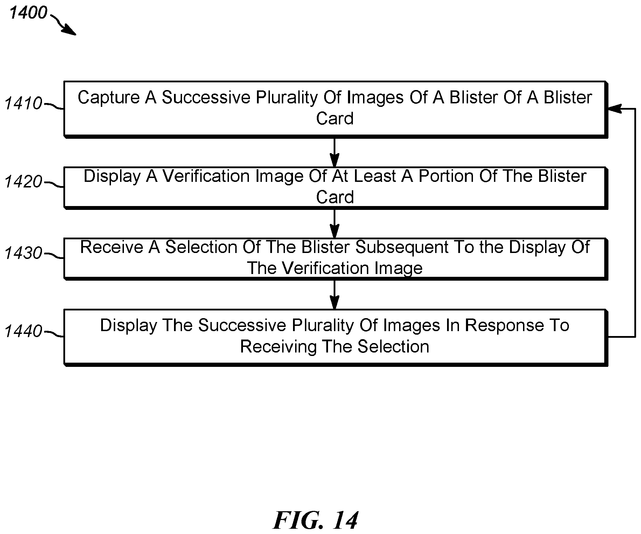

FIG. 14 is a flowchart illustrating one example method 1400 of verification for a blister card 100. The method 1400 is performed using the verification system 400 and can be used for verifying filling of the blister card 100 using the automatic blister card packager 200, another blister card packager, or when hand-filling. The method 1400 includes capturing, using the camera system 450, 460, a successive plurality of images of a blister of the blister card 100 (at block 1410). The successive plurality of images may be captured during filling of the blister card. For example, the successive plurality of images may depict the blister after successive layers of medications are dropped into the blister. In one example, capturing the successive plurality of images includes capturing the plurality of images from above the blister compartment 120 using the first verification camera system 450. In some examples, the electronic processor 410 controls the first verification camera system 450 to capture one or more images for each blister compartment 120 in a blister card 100. In some examples, the successive plurality of images of the blister compartments 120 is the collection of the one or more images captured for each blister compartment 120.

In some examples, the one or more images are captured after each medication is dropped in the blister compartment 120. For example, when a blister compartment 120 is prescribed to receive three pills, then three images are captured - one after each pill is dropped - of the blister compartment 120. In some examples, the one or more images are captured after each drop of pills in the blister compartment 120. The electronic processor 410 may divide dropping of the pills into the blister compartments 120 into multiple drops, each drop including one pill or more than one pill depending on, for example, the arrangement of the cartridges 245, traffic through the manifold 215, or the like. For example, a first drop may include one pill and a second drop may include three pills. The electronic processor 410 captures a first image of the one or more images after the first drop and a second image of the one or more images after the second drop. A person of ordinary skill in the art appreciates that the first image captured after the first drop is captured before the second drop is performed and so on.

In some implementations, the electronic processor 410 may be configured to capture the one or more images after each layer of medication is dropped. For example, the electronic processor 410 captures a first image after a first layer is dropped or added to the blister and a second image after a second layer is dropped or added to the blister. In some examples, the electronic processor 410 may determine that a layer of medication is added to the blister compartment 120, based on a number of pills added to the blister compartment 120, a size of one or more pills added to the blister compartment 120, dimensions (for example, length, and width) of the blister compartment 120, a combination of the foregoing, and the like. For example, a layer of medication may be added to the blister compartment when four small pills are added to the blister compartment 120 or when two large pills are added to the blister compartment 120. In some examples, after a first layer of medication is added to the blister compartment 120, a first image may be captured from above the blister compartment 120 using the first verification camera system 450 and, additionally, a second image may be captured from below the blister compartment 120 using the second verification camera system 460.

In some implementations, a user may select how often an image of a blister compartment is captured by selecting how many pills or a volume of pills that may be dropped into the blister compartment 120 without an image being captured. For example, a user may make a selection indicating that an image should be captured every 3 pills dropped in the blister compartment 120. In this example, an image of the blister compartment 120 is captured after three drops of one pill each are made, an image of the blister compartment 120 is captured after one drop of three pills is made, an image of the blister compartment 120 is captured after a first drop of one pill and a second drop of two pills, and an image of the blister compartment 120 is captured after a first drop of two pills and a second drop of one pill. In some examples, an image of the blister compartment 120 may be captured after the final drop is performed for the blister compartment 120 even if fewer than the selected number of pills have been dropped into or added to the blister compartment 120 since the last image was captured. For example, if the user selects 3 as the number of pills that may be dropped into a blister compartment without an image being captured, and only a single pill has been dropped into the blister compartment 120 since the last image was captured but the blister compartment 120 has received its prescribed number of pills (filling of the blister compartment 120 is complete), an image of the blister compartment 120 is captured.

In some examples, each blister compartment 120 included in a row of the blister card 100 may be filled simultaneously (or relatively simultaneously). In these examples, the electronic processor 410 may control the first verification camera system 450 to capture one or more images for each row of blister compartments 120 rather than for each blister compartment 120 and the successive plurality of images of the blister compartments 120 may be the collection of the one or more images captured for each row of blister compartments 120 included in the blister card 100. In some examples, the electronic processor 410 may divide an image of a row of blister compartments 120 into a plurality of images of individual blister compartments 120.

In other examples, each blister compartment 120 included in a column of the blister card 100 may be filled simultaneously (or relatively simultaneously). In these examples, the electronic processor 410 may control the first verification camera system 450 to capture one or more images for each column of blister compartments 120 rather than for each blister compartment 120 and the successive plurality of images of the blister compartments 120 may be the collection of the one or more images captured for each column of blister compartments 120 included in the blister card 100. In some examples, the electronic processor 410 may divide an image of a column of blister compartments 120into a plurality of images of individual blister compartments 120.

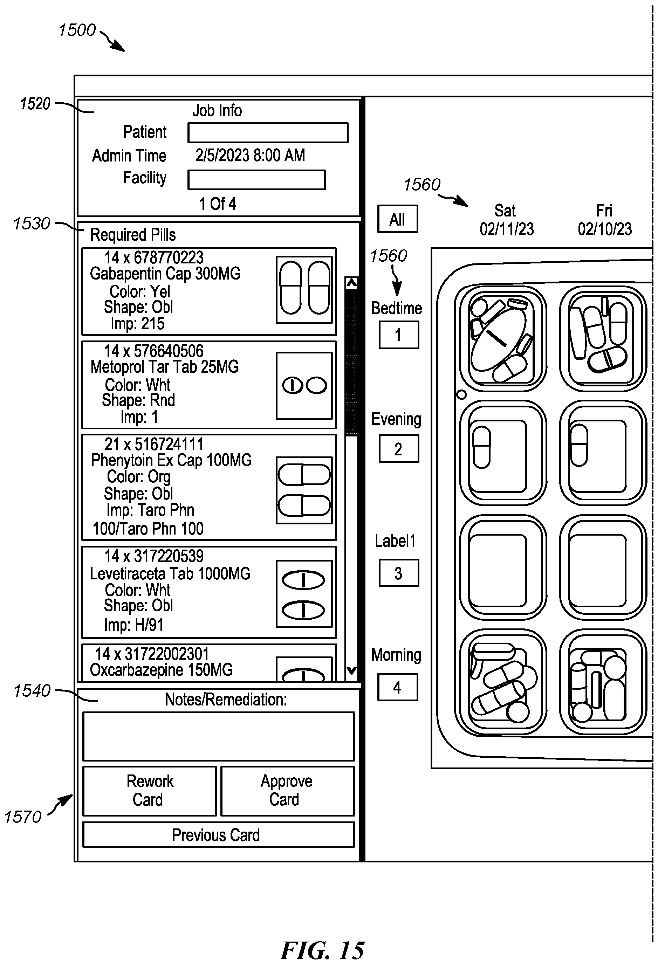

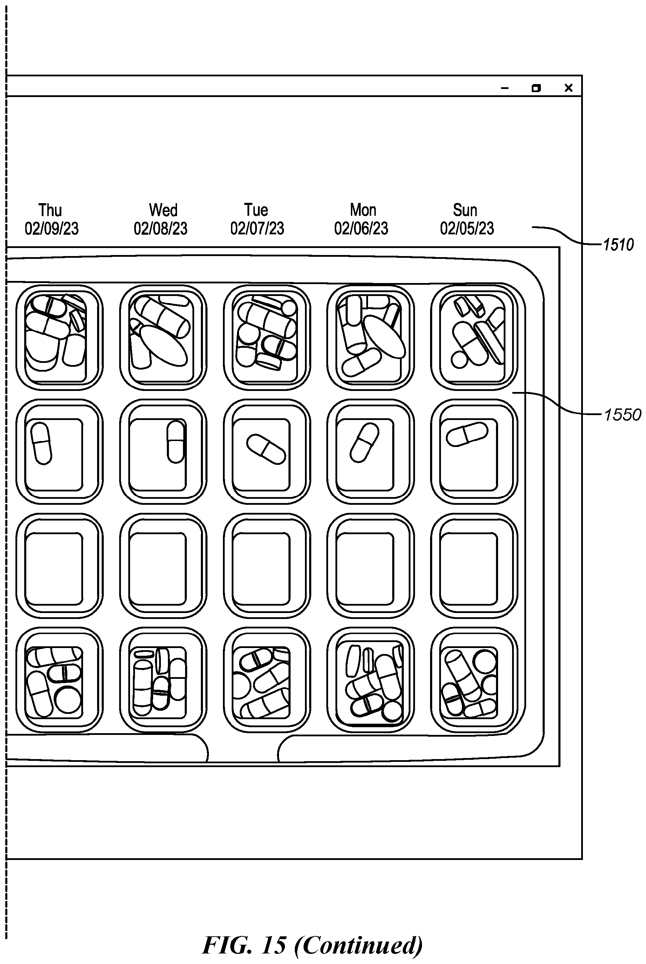

In some examples, the electronic processor 410 captures an image of the entire blister card 100 after filling, for example, after all the desired pills or prescribed medications are dropped into the respective blister compartments 120, but before sealing the blister card 100. The image of the filled blister card 100 may be a final image of the successive plurality of images captured. In one example, the image of the blister card 100 may be captured from below the blister card 100 (e.g., from below the blister compartments 120) using the second verification camera system 460. In some examples, each image of the successive plurality of images may be captured from the same orientation and displayed as illustrated in FIGS. 15-18.

In some examples, rather than capturing an image of the entire blister card 100 after filling, an image of the blister card 100 after filling may be created by the electronic processor 410 by combining images captured during the filling of the blister card 100 using known image processing techniques. For example, when a blister card 100 contains a plurality of blister compartments 120, for each blister compartment 120 of the blister card 100, an image of the filled blister compartment 120 may be utilized in creating an image of the filled blister card 100.

The method 1400 includes displaying, on a user interface, a verification image of at least a portion of the blister card (at block 1420). In some examples, the verification image is the image of the entire filled blister card 100 or a portion of the filled blister card 100. In one example, the display 490 is used to verify the filled blister cards 100. In other examples, a separate display, for example, of a separate device that is communicatively connected to the automatic blister card packager 200 or the verification system 400 is used to verify the filled blister cards 100. The electronic processor 410 generates a verification screen to display to a user for verification of the filled blister card 100. The verification image of the blister card 100 is displayed on the verification screen.

FIG. 15 illustrates one example verification screen 1500 generated by the electronic processor 410 for verifying the filled blister cards 100. In the example illustrated, the verification screen 1500 includes an image pane 1510, a job pane 1520, a reference pane 1530, and a navigation pane 1540. The image pane 1510 includes the image 1550 of the blister card 100 and labels 1560 associated with the image 1550 of the blister card 100. The labels 1560 include, for example, days and administration times to which the blister compartments 120 of the blister card 100 correspond. The labels 1560 are arrayed on the image pane 1510 such that each of the labels 1560 corresponds to a row or column of the blister card 100 in the image 1550 of the blister card 100. The labels 1560 may be selected to select a row or column of the blister compartments 120 of the blister card 100.

The job pane 1520 displays information relating to the prescription. For example, the job pane 1520 may display the name of the patient, the name of the facility, the number of the blister card 100, the packaging time, and/or the like. The reference pane 1530 displays information and/or images relating to the pills or medications packaged into the blister card 100 in the image 1550 of the blister card 100. For example, the reference pane 1530 may display reference images of the pills or medications, the NDC code, characteristics (e.g., shape, color, size, inscription, and/or the like), and/or the like of the pills or medications. Although not illustrated in FIG. 15 - FIG. 18, the reference pane 1530 may also display SIG codes associated with the pills or medications. A SIG code may inform a user (for example, a pharmacist) when and how often a pill or medication should be taken (for example, once a day, twice a day, in the evenings, or the like). The reference information relating to the pills may be retrieved from the national drug code (NDC) database or other publicly available or privately created drug databases. The navigation pane 1540 displays navigation buttons 1570 for navigating between the filled blister cards 100. In the example illustrated, the navigation buttons 1570 include an approve card button to approve the filled blister card 100, a previous card button to view the previously displayed blister card 100, and a rework card button to provide an instruction to repackage the blister card 100.

Returning to FIG. 14, the method 1400 includes receiving, via the user interface, a selection of the blister subsequent to the display of the verification image (at block 1430). In some examples, a selection of the blister may include the selection of a plurality of blisters (for example, one or more blister compartments 120 of the blister card 100). In some instances, all the pills within a blister compartment 120 may not be visible to a user from the image of the blister compartment 120. In these instances, the user may want to verify the filling of the blister compartment 120 using additional images. The user may select one or more blister compartments 120 in the image pane 1510 (see FIG. 15). In one example, the user may select (e.g., click on or hover over) one of the labels 1560 of the image pane 1510 to select a row or column of blister compartments 120. In another example, the user may select (e.g., click on or hover over) on a portion of the image 1550 of the blister card 100 corresponding to the desired blister compartment 120 (for example, over the blister compartment 120). In this example, the electronic processor 410 performs known image processing techniques to section the image 1550 of the blister card 100 according to the number of blister compartments 120 present in the image 1550. The electronic processor 410 assigns each section of the image 1550 to the blister compartment 120 shown in that section of the image 1550 such that when that section of the image 1550 is selected, the electronic processor 410 interprets the action as a selection of the blister compartment 120 shown in that section of the image 1550.

In some implementations, when a user hovers a cursor over a specific blister compartment 120, a pop-up window including what pills are expected in that blister compartment 120 may be displayed. For example, the electronic processor 410 may detect a hovering action over a blister. A hovering action may include, for example, determining that the cursor is on a portion of the screen for a specific amount of time, the cursor is being moved over a portion of the screen, or the like. The electronic processor 410 may then detect the blister associated with the portion of the screen. The electronic processor 410 generates the pop-up window including a list of expected pills that are expected in the blister. The pop-up window may be a temporary window that may be displayed only when the cursor is over the blister and may disappear when the cursor is moved away from the blister. The pop-up window may not be a separate window, but may include a pop-up box generated over the current window displaying the blisters. That is, the pop-up box may be displayed within or over the image pane 1510.

The method 1400 includes displaying, via the user interface, the successive plurality of images in response to receiving the selection (at block 1440). The successive plurality of images includes, for example, the one or more images captured for the blister at block 1410. In some examples, when the blister card 100 includes a greater number of blister compartments 120 than the number of blister compartments 120 selected, a subset of the plurality of images corresponding to the selected one or more blister compartments 120 is displayed via the user interface. The successive plurality of images (or subset thereof) may be displayed in various ways to show the selected blister as further explained below.

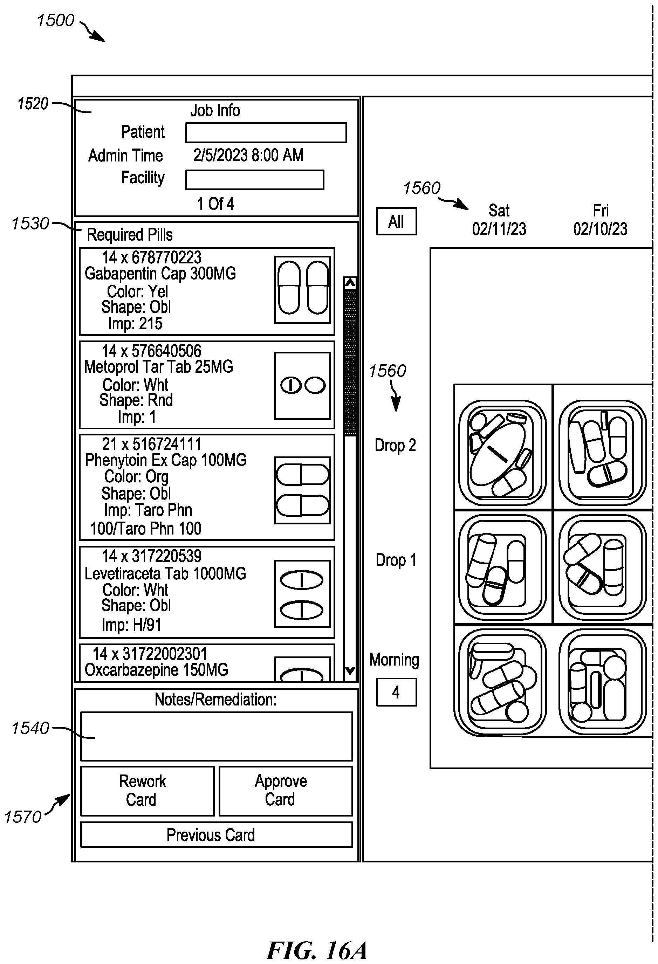

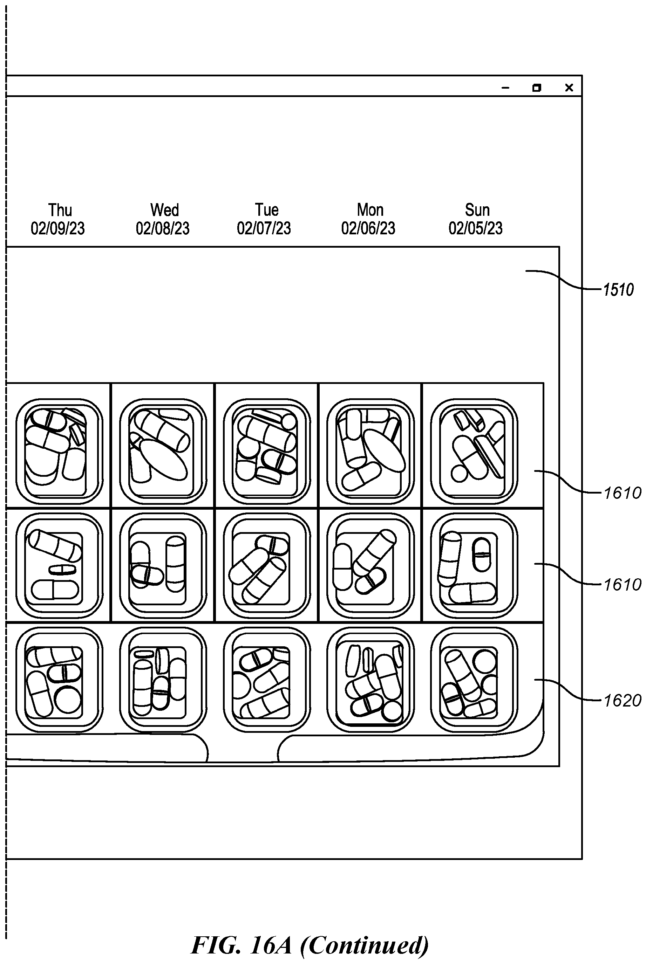

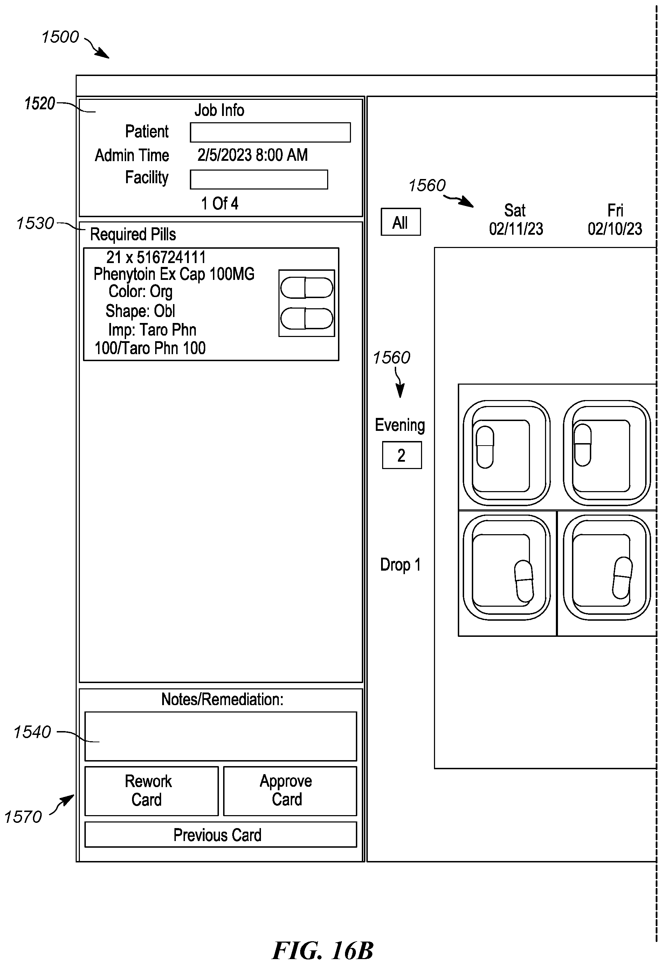

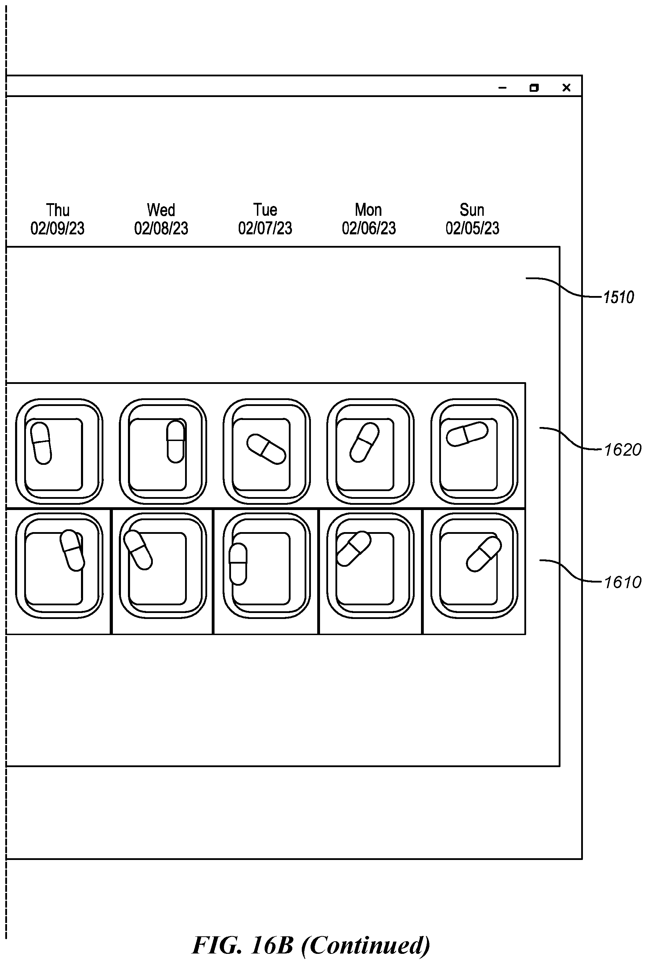

In the example of FIGS. 16A and 16B, the subset of a successive plurality of images 1610 corresponding to one or more selected blister compartments 120 is displayed in the image pane 1510 of the verification screen 1500. In this example, the image 1550 of the blister card 100 is cropped to show only the selected one or more blister compartments 120. The subset of the successive plurality of images 1610 corresponding to the selected one or more blister compartments 120 is displayed adjacent the cropped image 1620 of the selected one or more blister compartments 120. In one example, the subset of the successive plurality of images 1610 is displayed above (see FIG. 16A) or below (see FIG. 16B) the cropped image 1620. In some examples, the subset of the successive plurality of images are displayed to the left or right of the cropped image 1620. The subset of the successive plurality of images 1610 corresponding to a first blister compartment 120 and a portion of the cropped image 1620 corresponding to the first blister compartment 120 may be arrayed so that the first blister compartment 120 appears in the same row or same column of the combined display of the subset of the successive plurality of images 1610 and the cropped image 1620. In some examples, the successive plurality of images 1610 or the cropped images 1620 may be flipped vertically and/or horizontally (that is, mirrored) such that the pills in both the plurality of images 1610 and the cropped images 1620 are aligned with each other, for example, in the same orientation.

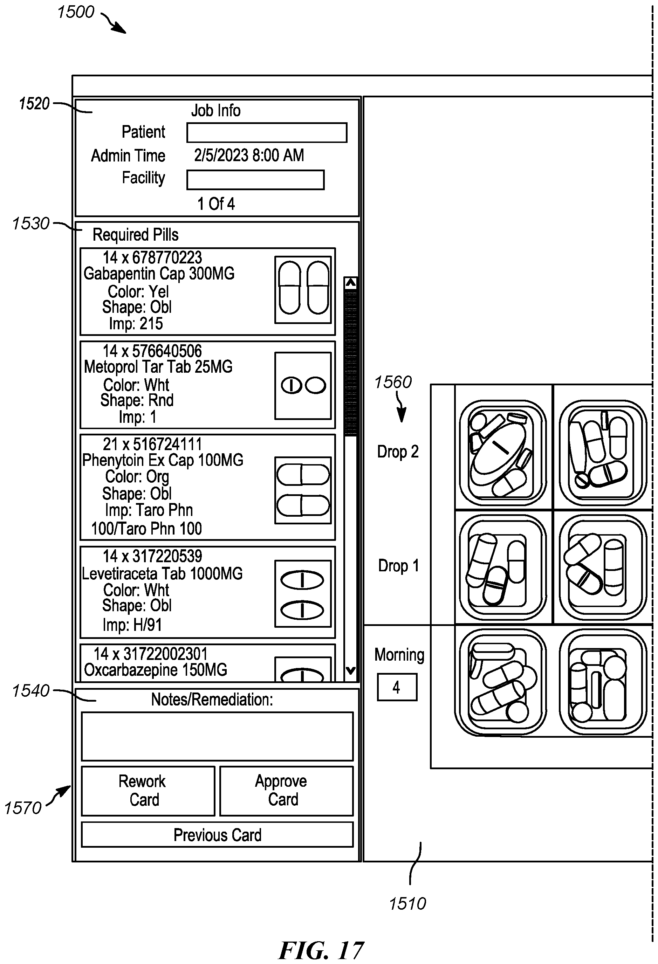

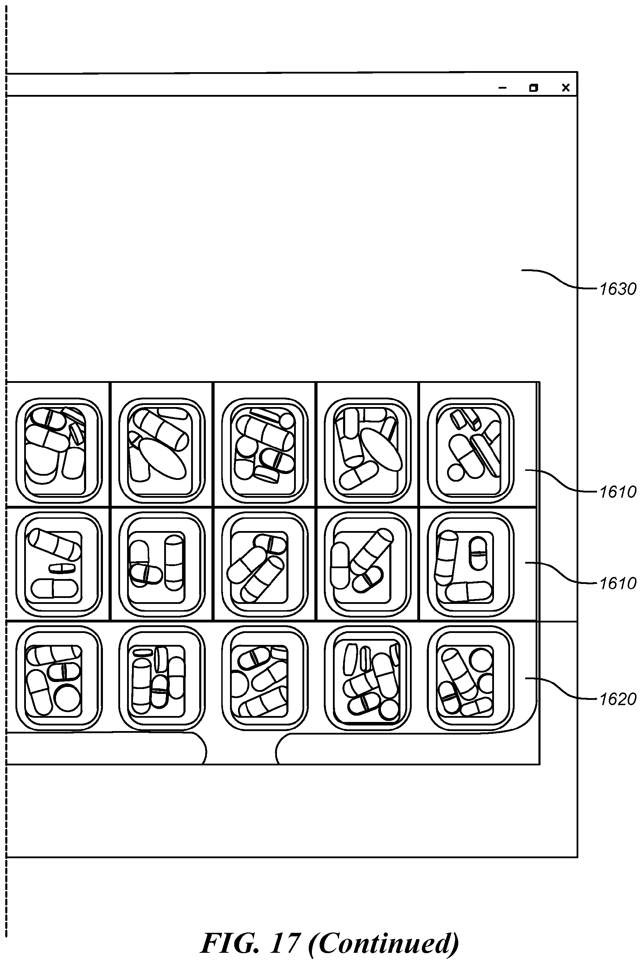

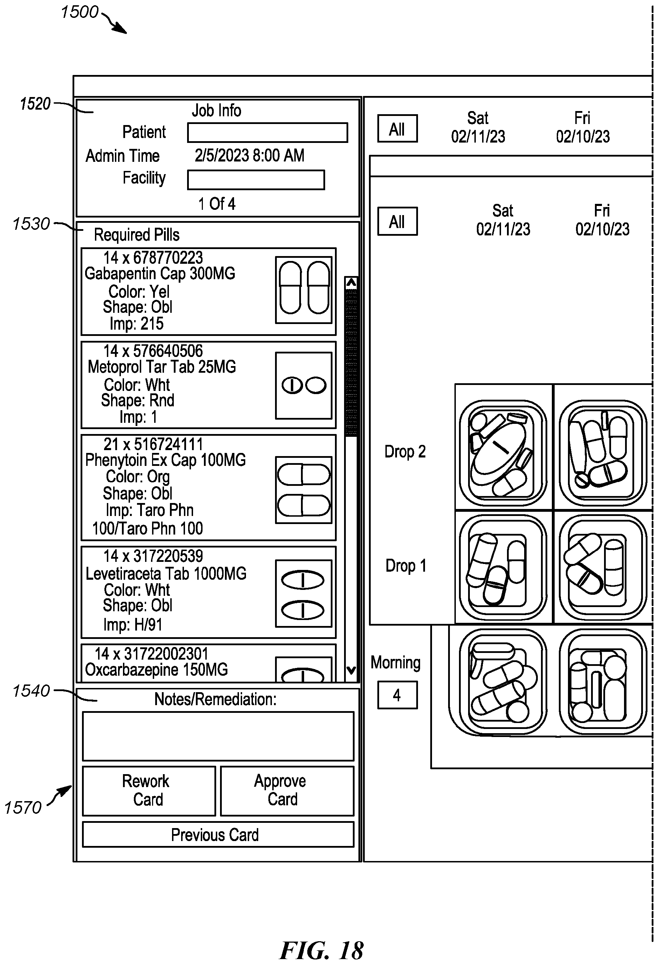

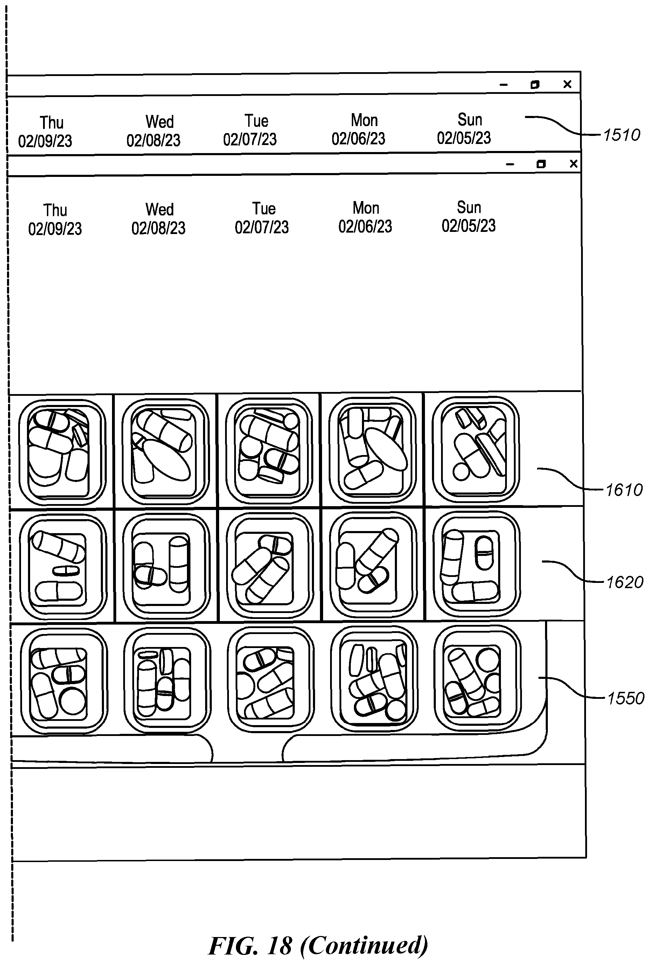

In the example of FIG. 17, the subset of the successive plurality of images 1610 may be displayed in a second image pane 1630 (for example, in the same window). The electronic processor 410 modifies the verification screen to add a second image pane 1630 adjacent (e.g., above, below, left of, or right of) the image pane 1510 (e.g., a first image pane). Even though the subset of the successive plurality of images 1610 is displayed in a separate pane, the subset of the successive plurality of images 1610 may be arrayed similarly as described with respect to FIGS. 16A and 16B. In the example of FIG. 18, the subset of the successive plurality of images 1610 may be displayed in a separate window. The separate window may be a new window or a pop-up window.

Thus, the present disclosure provides, among other things, verifications systems for automatic blister card packagers.

Certain embodiments of the invention are described in the following clauses:
Clause 1. A verification system for a blister card packaging system, the verification system comprising:
   an electronic processor, the electronic processor configured to:
   capture a successive plurality of images of a blister of a blister card;
   display a verification image of at least a portion of the blister card;
   receive a selection of the blister subsequent to the display of the verification image; and
   display the successive plurality of images in response to receiving the selection.
Clause 2. The verification system according to clause 1, wherein the successive plurality of images depict the blister after successive layers of medications are dropped into the blister and the electronic processor is further configured to:
   receive a selection of a volume of medication corresponding to each of the successive layers.
Clause 3. The verification system according to clause 1, wherein a final image of the successive plurality of images is captured after the blister is filled with a prescribed number of medications and before the blister card is sealed, wherein the verification image is the final image of the successive plurality of images.
Clause 4. The verification system according to clause 3, wherein the electronic processor is further configured to:
   generate a verification screen including an image pane, a job pane, a reference pane, and a navigation pane, wherein
   the verification image is displayed in the image pane along with a plurality of labels, each label of the plurality of labels corresponding to a row or column of the blister card in the verification image,
   for each medication included in the blister card, one or more reference images, a NDC code, one or more characteristics, and a SIG code are displayed in the reference pane, wherein the SIG code informs a user when the medication should be taken, how often the medication should be taken, or both, and
   one or more navigation buttons including at least one selected from the group consisting of an approve card button for approving the blister card, a previous card button for viewing a previously displayed blister card, and a rework card button for providing an instruction to repackage the blister card are displayed in the navigation pane.
Clause 5. The verification system according to clause 4, wherein the electronic processor is configured to receive a selection of the blister by:
   receiving a selection of one selected from the group consisting of a label of the plurality of labels corresponding to the blister and a portion of the verification image corresponding to the blister.
Clause 6. The verification system according to clause 1, wherein the electronic processor is configured to display the successive plurality of images in response to receiving the selection by:
   displaying the successive plurality of images adjacent to a cropped image of the blister card including only the blister so that a portion of the cropped image corresponding to the blister appears in a same row or a same column as the successive plurality of images corresponding to the blister.
Clause 7. The verification system according to clause 1, wherein the electronic processor is further configured to:
   detect a hovering action over the blister in the displayed image for verification; and
   generate a pop-up window including a list of medications that are expected in the blister.
Clause 8. A method for verification of a blister card, the method comprising:
   capturing a successive plurality of images of a blister of a blister card;
   displaying a verification image of at least a portion of the blister card;
   receiving a selection of the blister subsequent to the display of the verification image; and
   displaying the successive plurality of images in response to receiving the selection.
Clause 9. The method according to clause 8, wherein the successive plurality of images depict the blister after successive layers of medications are dropped into the blister and the method further comprising:
   receiving a selection of a volume of medication corresponding to each of the successive layers.
Clause 10. The method according to clause 8, wherein a final image of the successive plurality of images is captured after the blister is filled with a prescribed number of medications and before the blister card is sealed and the verification image is the final image of the successive plurality of images.
Clause 11. The method according to clause 10, the method further comprising:
   generating a verification screen including an image pane, a job pane, a reference pane, and a navigation pane, wherein
   the verification image is displayed in the image pane along with a plurality of labels, each label of the plurality of labels corresponding to a row or column of the blister card in the verification image,
   for each medication included in the blister card, one or more reference images, a NDC code, one or more characteristics, and a SIG code are displayed in the reference pane, wherein the SIG code informs a user when the medication should be taken, how often the medication should be taken, or both, and
   one or more navigation buttons including at least one selected from the group consisting of an approve card button for approving the blister card, a previous card button for viewing a previously displayed blister card, and a rework card button for providing an instruction to repackage the blister card are displayed in the navigation pane.
Clause 12. The method according to clause 11, wherein receiving a selection of the blister includes:
   receiving a selection of one selected from the group consisting of a label of the plurality of labels corresponding to the blister and a portion of the verification image corresponding to the blister.
Clause 13. The method according to clause 8, wherein displaying the successive plurality of images in response to receiving the selection includes:
   displaying the successive plurality of images adjacent to a cropped image of the blister card including only the blister so that a portion of the cropped image corresponding to the blister appears in a same row or a same column as the successive plurality of images corresponding to the blister.
Clause 14. The method according to clause 8, the method further comprising:
   detecting a hovering action over the blister in the displayed image for verification; and
   generating a pop-up window including a list of medications that are expected in the blister.
Clause 15. A blister card packaging system, the blister card packaging system comprising:
   an electronic processor, the electronic processor configured to:
   capture a successive plurality of images of a blister of a blister card;
   display a verification image of at least a portion of the blister card;
   receive a selection of the blister subsequent to the display of the verification image; and
   display the successive plurality of images in response to receiving the selection.
Clause 16. The blister card packaging system according to clause 15, wherein the successive plurality of images depict the blister after successive layers of medications are dropped into the blister and the electronic processor is further configured to:
   receive a selection of a volume of medication corresponding to each of the successive layers.
Clause 17. The blister card packaging system according to clause 15, wherein a final image of the successive plurality of images is captured after the blister is filled with a prescribed number of medications and before the blister card is sealed and the verification image is the final image of the successive plurality of images.
Clause 18. The blister card packaging system according to clause 17, wherein the electronic processor is further configured to:
   generate a verification screen including an image pane, a job pane, a reference pane, and a navigation pane, wherein
   the verification image is displayed in the image pane along with a plurality of labels, each label of the plurality of labels corresponding to a row or column of the blister card in the verification image,
   for each medication included in the blister card, one or more reference images, a NDC code, one or more characteristics, and a SIG code are displayed in the reference pane, wherein the SIG code informs a user when the medication should be taken, how often the medication should be taken, or both, and
   one or more navigation buttons including at least one selected from the group consisting of an approve card button for approving the blister card, a previous card button for viewing a previously displayed blister card, and a rework card button for providing an instruction to repackage the blister card are displayed in the navigation pane.
Clause 19. The blister card packaging system according to clause 18, wherein the electronic processor is configured to receive a selection of the blister by:
   receiving a selection of one selected from the group consisting of a label of the plurality of labels corresponding to the blister and a portion of the verification image of corresponding to the blister.
Clause 20. The blister card packaging system according to clause 15, wherein the electronic processor is configured to display the successive plurality of images in response to receiving the selection by:
   displaying the successive plurality of images adjacent to a cropped image of the blister card including only the blister so that a portion of the cropped image corresponding to the blister appears in a same row or a same column as the successive plurality of images corresponding to the blister.

## Claims

1. A verification system for a blister card packaging system, the verification system comprising:
an electronic processor, the electronic processor configured to:
capture a successive plurality of images of a blister of a blister card;
display a verification image of at least a portion of the blister card;
receive a selection of the blister subsequent to the display of the verification image; and
display the successive plurality of images in response to receiving the selection.

2. The verification system according to claim 1, wherein the successive plurality of images depict the blister after successive layers of medications are dropped into the blister and the electronic processor is further configured to:
receive a selection of a volume of medication corresponding to each of the successive layers.

3. The verification system according to claim 1, wherein a final image of the successive plurality of images is captured after the blister is filled with a prescribed number of medications and before the blister card is sealed, wherein the verification image is the final image of the successive plurality of images.

4. The verification system according to claim 3, wherein the electronic processor is further configured to:
generate a verification screen including an image pane, a job pane, a reference pane, and a navigation pane, wherein
the verification image is displayed in the image pane along with a plurality of labels, each label of the plurality of labels corresponding to a row or column of the blister card in the verification image,
for each medication included in the blister card, one or more reference images, a NDC code, one or more characteristics, and a SIG code are displayed in the reference pane, wherein the SIG code informs a user when the medication should be taken, how often the medication should be taken, or both, and
one or more navigation buttons including at least one selected from the group consisting of an approve card button for approving the blister card, a previous card button for viewing a previously displayed blister card, and a rework card button for providing an instruction to repackage the blister card are displayed in the navigation pane, and optionally:
wherein the electronic processor is configured to receive a selection of the blister by:
receiving a selection of one selected from the group consisting of a label of the plurality of labels corresponding to the blister and a portion of the verification image corresponding to the blister.

5. The verification system according to claim 1, wherein the electronic processor is configured to display the successive plurality of images in response to receiving the selection by:
displaying the successive plurality of images adjacent to a cropped image of the blister card including only the blister so that a portion of the cropped image corresponding to the blister appears in a same row or a same column as the successive plurality of images corresponding to the blister, or
wherein the electronic processor is further configured to:
detect a hovering action over the blister in the displayed image for verification; and
generate a pop-up window including a list of medications that are expected in the blister.

6. A method for verification of a blister card, the method comprising:
capturing a successive plurality of images of a blister of a blister card;
displaying a verification image of at least a portion of the blister card;
receiving a selection of the blister subsequent to the display of the verification image; and
displaying the successive plurality of images in response to receiving the selection.

7. The method according to claim 6, wherein the successive plurality of images depict the blister after successive layers of medications are dropped into the blister and the method further comprising:
receiving a selection of a volume of medication corresponding to each of the successive layers.

8. The method according to claim 6, wherein a final image of the successive plurality of images is captured after the blister is filled with a prescribed number of medications and before the blister card is sealed and the verification image is the final image of the successive plurality of images.

9. The method according to claim 8, the method further comprising:
generating a verification screen including an image pane, a job pane, a reference pane, and a navigation pane, wherein
the verification image is displayed in the image pane along with a plurality of labels, each label of the plurality of labels corresponding to a row or column of the blister card in the verification image,
for each medication included in the blister card, one or more reference images, a NDC code, one or more characteristics, and a SIG code are displayed in the reference pane, wherein the SIG code informs a user when the medication should be taken, how often the medication should be taken, or both, and
one or more navigation buttons including at least one selected from the group consisting of an approve card button for approving the blister card, a previous card button for viewing a previously displayed blister card, and a rework card button for providing an instruction to repackage the blister card are displayed in the navigation pane, and optionally:
wherein receiving a selection of the blister includes:
receiving a selection of one selected from the group consisting of a label of the plurality of labels corresponding to the blister and a portion of the verification image corresponding to the blister.

10. The method according to claim 6, wherein displaying the successive plurality of images in response to receiving the selection includes:
displaying the successive plurality of images adjacent to a cropped image of the blister card including only the blister so that a portion of the cropped image corresponding to the blister appears in a same row or a same column as the successive plurality of images corresponding to the blister, or
the method further comprising:
detecting a hovering action over the blister in the displayed image for verification; and
generating a pop-up window including a list of medications that are expected in the blister.

11. A blister card packaging system, the blister card packaging system comprising:
an electronic processor, the electronic processor configured to:
capture a successive plurality of images of a blister of a blister card;
display a verification image of at least a portion of the blister card;
receive a selection of the blister subsequent to the display of the verification image; and
display the successive plurality of images in response to receiving the selection.

12. The blister card packaging system according to claim 11, wherein the successive plurality of images depict the blister after successive layers of medications are dropped into the blister and the electronic processor is further configured to:
receive a selection of a volume of medication corresponding to each of the successive layers.

13. The blister card packaging system according to claim 11, wherein a final image of the successive plurality of images is captured after the blister is filled with a prescribed number of medications and before the blister card is sealed and the verification image is the final image of the successive plurality of images.

14. The blister card packaging system according to claim 13, wherein the electronic processor is further configured to:
generate a verification screen including an image pane, a job pane, a reference pane, and a navigation pane, wherein
the verification image is displayed in the image pane along with a plurality of labels, each label of the plurality of labels corresponding to a row or column of the blister card in the verification image,
for each medication included in the blister card, one or more reference images, a NDC code, one or more characteristics, and a SIG code are displayed in the reference pane, wherein the SIG code informs a user when the medication should be taken, how often the medication should be taken, or both, and
one or more navigation buttons including at least one selected from the group consisting of an approve card button for approving the blister card, a previous card button for viewing a previously displayed blister card, and a rework card button for providing an instruction to repackage the blister card are displayed in the navigation pane, and optionally:
wherein the electronic processor is configured to receive a selection of the blister by:
receiving a selection of one selected from the group consisting of a label of the plurality of labels corresponding to the blister and a portion of the verification image of corresponding to the blister.

15. The blister card packaging system according to claim 11, wherein the electronic processor is configured to display the successive plurality of images in response to receiving the selection by:
displaying the successive plurality of images adjacent to a cropped image of the blister card including only the blister so that a portion of the cropped image corresponding to the blister appears in a same row or a same column as the successive plurality of images corresponding to the blister.
